(19) Europäisches Patentamt
European Patent Office
Office européen des brevets

(11) EP 1 532 116 B1

(12) EUROPÄISCHE PATENTSCHRIFT

(45) Veröffentlichungstag und Bekanntmachung des Hinweises auf die Patenterteilung:
**16.09.2009 Patentblatt 2009/38**

(21) Anmeldenummer: **03720484.9**

(22) Anmeldetag: **16.04.2003**

(51) Int Cl.:
***C07D 231/14*** (2006.01) ***C07D 231/16*** (2006.01)

(86) Internationale Anmeldenummer:
**PCT/EP2003/004000**

(87) Internationale Veröffentlichungsnummer:
**WO 2003/104201 (18.12.2003 Gazette 2003/51)**

(54) **1H-PYRAZOL-DERIVATE, VERFAHREN ZU IHRER HERSTELLUNG UND DEREN VERWENDUNG ALS NITRIFIKATIONSINHIBITOREN**

1H-PYRAZOL DERIVATIVES, METHOD FOR PRODUCING THE SAME AND THE USE THEREOF AS NITRIFICATION INHIBITORS

DERIVES DE 1H-PYRAZOLE, LEUR PROCEDE DE PRODUCTION ET LEUR UTILISATION EN TANT QU'INHIBITEURS DE NITRIFICATION

(84) Benannte Vertragsstaaten:
**AT BE BG CH CY CZ DE DK EE ES FI FR GB GR HU IE IT LI LU MC NL PT RO SE SI SK TR**

(30) Priorität: **05.06.2002 DE 10224823**

(43) Veröffentlichungstag der Anmeldung:
**25.05.2005 Patentblatt 2005/21**

(73) Patentinhaber: **SKW STICKSTOFFWERKE PIESTERITZ GmbH**
**06886 Lutherstadt Wittenberg (DE)**

(72) Erfinder:
- **RADICS, Ute**
  **06888 Dabrun (DE)**
- **NICLAS, Hans-Joachim**
  **12435 Berlin (DE)**
- **NIENDORF, Klaus**
  **06886 Wittenberg (DE)**
- **MICHEL, Hans-Jürgen**
  **04827 Machern (DE)**
- **GRABARSE, Margit**
  **04687 Seelingstädt (DE)**
- **FUCHS, Michael**
  **04451 Cunnersdorf (DE)**

(74) Vertreter: **Schneider, Michael et al**
**Hammonds**
**Karl-Scharnagel-Ring 7**
**80539 München (DE)**

(56) Entgegenhaltungen:
**DE-A- 10 117 821** **DE-A- 10 135 423**

Bemerkungen:
Die Akte enthält technische Angaben, die nach dem Eingang der Anmeldung eingereicht wurden und die nicht in dieser Patentschrift enthalten sind.

EP 1 532 116 B1

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

Printed by Jouve, 75001 PARIS (FR)

## Beschreibung

Die vorliegende Erfindung betrifft 1 H-Pyrazol-Derivate, Verfahren zu ihrer Herstellung, Düngemittelzusammensetzungen, die sie umfassen, sowie deren Verwendung zur Hemmung bzw. Steuerung der Nitrifikation.

Die Anwendung von Stickstoffdüngemitteln, die Nitrifikationsinhibitoren enthalten, ist dadurch besonders vorteilhaft, dass die im allgemeinen rasch ablaufende Nitrifikation, d. h. die mikrobielle Umwandlung von Amid- und Ammoniumstickstoff zu Nitratstickstoff, auf diese Weise verzögert wird. Die derart gesteuerte Nitratbildung im Boden vermeidet eine zu intensive Nitratbildung und damit die Akkumulation von Nitrat. Eine so bewirkte Zurückdrängung des sonst entstehenden Nitratüberschusses im Boden konserviert den Düngerstickstoff in Form von Ammoniumionen, sorgt damit für eine höhere Düngereffizienz und führt gleichzeitig zu einer erheblich verringerten Umweltbelastung im Rahmen einer Stickstoffdüngung.

So ist bekannt, dass Nitrat im Gegensatz zum Ammonium im Boden nur äußerst schlecht sorbiert wird und demzufolge der Auswaschung bzw. der Verlagerung in tiefere Bodenschichten unterliegt. Andererseits führt die infolge einer Nitratanreicherung im Boden verstärkt einsetzende Denitrifikation zu gasförmigen Stickstoffverlusten, wobei neben molekularem Stickstoff auch solche Oxide wie Stickstoffmonoxid bzw. Lachgas emittiert werden, die zur Versauerung des Bodens beitragen bzw. als klimaverändernde Gase bekannt sind. Gleichzeitig wird einer Nitratanreicherung im Grundwasser entgegengewirkt.

Gerade in der Reduzierung der beschriebenen Verlustpotentiale liegt der wirtschaftliche Vorteil von mit Nitrifikationsinhibitoren versehenen Stickstoffdüngemitteln, der sich u. a. in der erhöhten Nährstoffeffizienz dieser Dünger niederschlägt. Um das zu unterstreichen, sei erwähnt, dass N-Verluste von konventionellen Stickstoffdüngern durchaus unter ungünstigen Bedingungen Größenordnungen von bis zu 40 % und mehr annehmen können.

Als wirksame Nitrifikationshemmer wurden unter anderem stickstoffhaltige heterocyclische Verbindungen, bevorzugt aus der Reihe der 1H-Pyrazole und der 1,2,4-Triazole, vorgeschlagen (vgl. z. B. US 3,635,690, US 4,969,946, US 4,523,940, EP-A 166 420, JP-OS 71-04135, US 3,697,244).

Ein wesentlicher Nachteil der meistens sehr gut nitrifikationshemmend wirkenden Pyrazol-Derivate besteht in ihrer relativ hohen Flüchtigkeit bzw. besonders bei am N-1-Stickstoffatom substituierten Verbindungen in ihrer Hydrolyseempfindlichkeit, in deren Folge zwar noch wirksame, aber wieder flüchtige Derivate gebildet werden. Aus diesen Gründen lässt sich eine Anwendung bei festen Düngemitteln, insbesondere in Kombinationen mit Harnstoff, nicht ohne weiteres realisieren. Zur Beseitigung dieses Mangels sind Vorschläge zur chemischen Abwandlung der 1-N-unsubstituierten Basispyrazole bekannt (DE-OS 27 45 833, EP-A 508 191, DD 292 232, DE-OS 40 18 395, EP-A 160 804, DE-OS 37 04 359, SU 1 470 732, DE-OS 44 46 194, EP-A 808 297). Mit dem Rückgang der Flüchtigkeit durch Derivatisierung ist jedoch in der Regel zugleich auch ein Abfall in der Wirkung als Nitrifikationsinhibitor verbunden.

Andere Lösungen zur Reduzierung der Flüchtigkeit von nitrifikationshemmena wirkenden Pyrazolen bestehen in der Ausnutzung des eigentlich bekannten Prinzips der Salzbildung mit Säuren (EP-A 529 473, WO 98/05 607, DE-OS 40 18 395).

Sowohl die mineralsauren Salze der nitrifiziden Basispyrazole als auch nitrifizid wirkende Pyrazole, die am 1-N-Stickstoff hydrophile Gruppen tragen, können als Nitrifikationsinhibitoren noch nicht voll befriedigen, da sie eine zu geringe Hydrolysestabilität besitzen, wodurch die Wirkungsdauer im Boden und die Lagerstabilität herabgesetzt ist. Dies betrifft z. B. N-Glyoxylpyrazole (DE-OS 37 04 359) oder N-Hydroxymethylpyrazole (SU-A 1 470 737). Mit der Einführung von verschiedenartigen lipophilen Gruppen in 1-N-Position (DE-OS 101 17 821, DE-OS 101 35 423) konnte zwar die Hydrolyseanfälligkeit dieser Pyrazol-Derivate beseitigt werden, jedoch sind diese Nitrifikationsinhibitoren bei niedrigen Konzentrationen nicht mehr ausreichend lang leistungsfähig.

Der vorliegenden Erfindung lag die Aufgabe zugrunde, Wirkstoffe zur Hemmung bzw. Steuerung der Nitrifikation von Ammoniumstickstoff zu entwickeln, die aufgrund ihrer geringen Flüchtigkeit und ihrer Hydrolysestabilität für alle Applikationsverfahren geeignet sind, aber auch alle Möglichkeiten zur Einarbeitung in oder Aufbringung auf mineralische stickstoffhaltige Dünger zulassen und außerdem eine ausgezeichnete Residualwirkung aufweisen.

Diese Aufgabe wurde erfindungsgemäß durch die 1H-Pyrazol-Derivate entsprechend Formel (I) gelöst.

Es hat sich nämlich gezeigt, dass diese Verbindungen neben der guten nitrifikationshemmenden Wirkung den außerordentlichen Vorteil einer sehr geringen Hydrolyseneigung besitzen, wodurch Wirkstoffverluste bei der Oberflächenapplikation, aber auch bei der Lagerung von Dünger-Wirkstoff-Gemischen nahezu ausgeschlossen sind. Im Gegensatz zu bereits bekannten Pyrazol-Derivaten liegt der besondere Vorteil der erfindungsgemäßen Verbindungen außerdem darin, dass sie nicht nur problemlos mit vielen "nitrathaltigen" N-Düngemitteln wie Ammonsulfatsalpeter und sauer reagierenden Düngern wie beispielsweise Ammoniumsulfat kombiniert werden können, ohne dass sie vorher in ihre Salze überführt werden müssen, um Wirkstoffverluste infolge Flüchtigkeit zu verhindern, sondern aufgrund der beschriebenen Eigenschaften verlustfrei mit harnstoffbasierten Düngemitteln formuliert werden können. Dies ist deshalb so bemerkenswert, da bekannt ist, dass Harnstoff stets mit geringen Anteilen von freiem Ammoniak begleitet ist, der die sonst schwächer basischen Pyrazol-Derivate aus ihrem für die Wirkung unerlässlichen Verbund mit Harnstoff verdrängt und somit zu unterkritischen Konzentrationen an Nitrifikationsinhibitor führt, wodurch eine ausreichende Nitrifikationshemmung nicht gewährleistet ist.

Außerdem sind die erfindungsgemäßen 1H-Pyrazol-Derivate durch ihre Lipophilie und dem damit verbundenen hydrophoben Charakter für eine Aufbringung auf die Düngeroberfläche sehr gut geeignet.

Schließlich kommt die nitrifizide Wirkung der erfindungsgemäßen 1H-Pyrazol-Derivate auch bei sehr geringen Wirkstoffkonzentrationen zur vollen Entfaltung, was ebenfalls nicht vorhersehbar war.

Die erfindungsgemäß verwendeten 1H-Pyrazol-Derivate entsprechen der allgemeinen Formel (I)

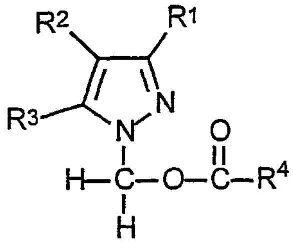

(I)

wobei
$R^1$ für einen $C_1$-$C_4$-Alkylrest oder $C_3$-$C_8$-Cycloalkylrest,
$R^2$ für H, Halogen (F, Cl, Br, I), einen $C_1$-$C_4$-Alkylrest oder $C_3$-$C_8$-Cycloalkylrest,
$R^3$ für H und
$R^4$ für $C_1$-$C_{20}$-Alkyl, $C_3$-$C_8$-Cycloalkyl, $C_6$-$C_{10}$-Aryl oder Alkylaryl mit $C_1$-$C_4$-Alkyl-und $C_6$-$C_{10}$-Arylgruppen steht, wobei die Alkyl-und Arylreste von $R^4$ durch eine Halogen-, Hydroxyl-, Trimethylsilyl-, Amino-, Nitro-, Cyano-, Carbonyl-, Carboxyl- oder eine Carboxyalkylgruppe mit 1 bis 5 C-Atomen substituiert sein können.

Die $C_1$- bis $C_4$-Alkylreste $R^1$ und $R^2$ können linear oder verzweigt sein. Vorzugsweise stellen $R^1$ und $R^2$ unabhängig voneinander einen Methylrest oder $R^1$ = Methyl und $R^2$ = Wasserstoff oder Chlor dar.

Die erfindungsgemäßen Verbindungen zeichnen sich durch ihren lipophilen Charakter und durch ausgezeichnete nitrifizide Eigenschaften auch bei geringen Aufwandmengen aus. Die Einführung des Restes -C(O)$R^4$ führt zu einem erheblichen Anstieg der Hydrolysestabilität der Verbindungen, ohne dass ein Rückgang der nitrifiziden Wirkung erfolgt, teilweise wird diese sogar noch verstärkt. Aufgrund dieser Eigenschaften sind die erfindungsgemäßen 1H-Pyrazol-Derivate bevorzugt für eine Oberflächenapplikation auf den Düngemittelgranulaten geeignet.

Die erfindungsgemäß vorgeschlagenen 1H-Pyrazol-Derivate (I) (wobei $R^1$, $R^2$, $R^3$ und $R^4$ die oben genannte Bedeutung besitzen) sind aus dem jeweiligen Hydroxymethyl-pyrazol (II) durch Reaktion mit Carbonsäurechloriden gemäß der allgemeinen Gleichung (1) zugänglich, wobei man das entsprechende Hydroxymethyl-pyrazol mit dem Carbonsäurechlorid (III) im bevorzugten Molverhältnis von 1 : 1,0 bis 1,5 gegebenenfalls unter Zusatz von 1 bis 100 Mol % eines Acylierungskatalysators wie beispielsweise 4-Dimethylamino-pyridin (DMAP) oder Pyridin unter Zusatz 1,0 bis 2 mol einer Base wie z. B. Pyridin, Triethylamin oder Kaliumcarbonat in einem organischen Lösemittel ausgewählt aus der Gruppe Halogenkohlenwasserstoffe (wie z. B. Methylenchlorid), Kohlenwasserstoffe (beispielsweise Toluol) oder trockenem Pyridin bei Temperaturen von - 15 bis 100 °C, vorzugsweise bei - 10 bis + 10 °C, umsetzt.

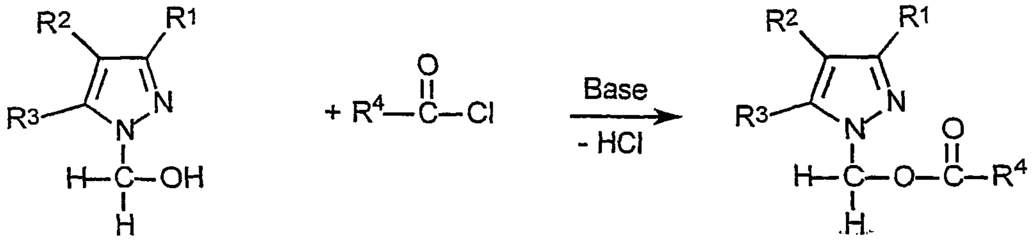


(II) (III) (I) (1)

Die 1H-Pyrazol-Derivate (I) (wobei $R^1$, $R^2$, $R^3$ und $R^4$ die oben genannte Bedeutung besitzen) können auch erhalten werden, indem man die Hydroxymethyl-pyrazole (II) (wobei $R^1$, $R^2$, $R^3$ die oben genannte Bedeutung besitzen) mit Carbonsäureanhydriden (III) gemäß der allgemeinen Gleichung (2) umsetzt, wobei das entsprechende Hydroxymethyl-pyrazol (II) mit dem Carbonsäureanhydrid (IV) im bevorzugten Molverhältnis von 1 : 1,0 bis 2,0 gegebenenfalls unter

Zusatz von 0,2 bis 10 Mol % 4-Dimethylamino-pyridin (DMAP) und/oder unter Zusatz von molaren Mengen Triethylamin in einem organischen Lösemittel ausgewählt aus der Gruppe Halogenkohlenwasserstoffe (wie z. B. Methylenchlorid), aliphatische Ether (wie z. B. Diethylether) oder der entsprechenden Carbonsäuren $R^4$-COOH (wie z. B. Essigsäure) bei Temperaturen von - 5 bis 80 °C zur Reaktion gebracht wird. Besonders bevorzugt wird bei einer Reaktionstemperatur von 50 bis 100 °C gearbeitet, wenn als Lösemittel die entsprechende Carbonsäure $R^4$-COOH zum Einsatz kommt.

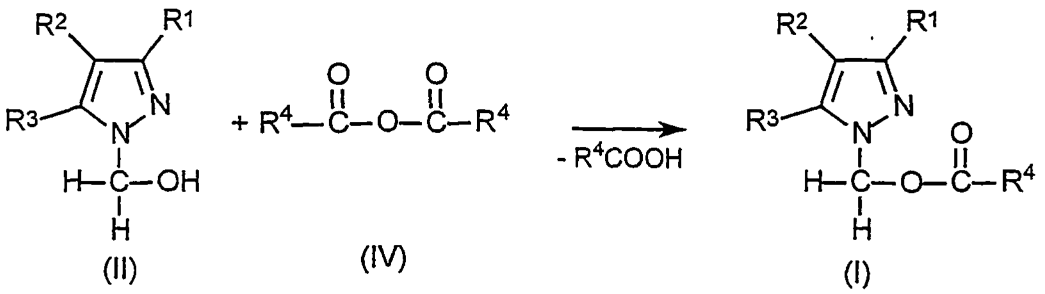


(II) (IV) (I)

(2)

Die Hydroxymethyl-pyrazole (II) bzw. deren Herstellung sind aus der Literatur bekannt (J. Hüttel Chem. Ber. 85 (1952) 820, OS 28 35 158, OS 199 13 475).

Die Reaktionsprodukte fallen je nach Substituentenmuster als destillierbare Flüssigkeiten oder kristalline Substanzen an. Bei der Synthese der erfindungsgemäßen Verbindungen entsprechend den Gleichungen (1) und (2) wurde das Auftreten von Isomeren in Bezug auf die Stellung des $R^1$- und $R^3$-Substituenten außer Acht gelassen. Es wurde von vornherein von regioisomeren Mischungen der Hydroxymethyl-pyrazole (II) ausgegangen. Die Bildung von optischen Isomeren wurde bei der Herstellung der Hydroxymethyl-pyrazole (II) ebenfalls außer Acht gelassen. Falls gewünscht, können die jeweiligen Isomeren jedoch mittels der üblichen, dem Fachmann bekannten Verfahren getrennt werden.

Ein weiterer Gegenstand der vorliegenden Erfindung ist die Verwendung der erfindungsgemäßen 1H-Pyrazol-Derivate zur Hemmung bzw. Regelung der Nitrifikation.

Außerdem betrifft die vorliegende Erfindung eine Zusammensetzung umfassend die erfindungsgemäßen 1H-Pyrazol-Derivate sowie ggf. weitere übliche Nitrifikationsinhibitoren und/oder Ureasehemmstoffe in Kombination mit festen oder flüssigen ammonium- und/oder amidhaltigen Düngemitteln. Als Stickstoffdüngemittel werden vorzugsweise Ammoniumsalze, wie z. B. Kalkammonsalpeter, Ammonsulfatsalpeter, Ammoniumnitrat oder Harnstoff eingesetzt. Aufwandmengen von 0,01 bis 10,0 Gewichtsprozent, insbesondere 0,1 bis 3,0 Gewichtsprozent, berechnet auf den reduzierten Stickstoffgehalt des Düngers, d. h. auf Ammonium- und Carbamid-Stickstoff, genügen für den praktischen Einsatz.

Die Art und Weise, wie die erfindungsgemäß vorgeschlagenen Nitrifikationsinhibitoren mit dem Düngemittel kombiniert werden, ist relativ unkritisch. So können die 1H-Pyrazol-Derivate bevorzugt allein oder im Gemisch mit anderen Zusatzstoffen als Lösung auf die Oberfläche der Düngemittelgranulate aufgebracht werden, wobei das Lösemittel (vorzugsweise aprotische Lösemittel, wie z. B. Ester, Öle, Ketone, Ether etc.) durch Trocknung entfernt wird.

Alternativ hierzu können die 1H-Pyrazol-Derivate allein oder im Gemisch mit anderen Zusatzstoffen in die Düngemittelschmelze oder -slurry während des Formgebungsprozesses eingebracht werden, wobei sie sehr homogen im Dünger verteilt vorliegen.

Die nachfolgenden Beispiele sollen die Erfindung näher veranschaulichen.

Beispiele

Darstellung von 1H-Pyrazol-Derivaten (I)

**Variante A:**

**Allgemeine Synthesevorschrift:**

0,25 mol Hydroxymethyl-pyrazol (II) und 0,025 mol 4-Dimethylamino-pyridin (DMAP) werden in 200 ml trockenem Pyridin gelöst und auf 5 °C abgekühlt. Unter Rühren und Eiskühlung werden 0,3 mol Carbonsäurechlorid (III) so zugetropft, dass die Temperatur nicht über 5 °C steigt. Man lässt 2 h bei 5 bis 10 °C nachrühren und anschließend bei Raumtemperatur über Nacht stehen. Die Reaktionsmischung wird auf Eis gegeben und mit Diethylether extrahiert. Man wäscht die organische Phase mit 5 Gew.-%iger Salzsäure mit gesättigter Natriumchloridlösung, mit 5 Gew.-%iger Soda- und nochmals mit Kochsalzlösung. Die organische Phase wird getrocknet und das Lösemittel unter Vakuum verdampft. Das Rohprodukt wird durch Umkristallisieren oder durch Säulenchromatographie gereinigt.

**Variante B:**

**Allgemeine Synthesevorschrift:**

0,25 mol Hydroxymethyl-pyrazol (II), 0,01 mol 4-Dimethylamino-pyridin (DMAP) und 0,25 mol Pyridin werden in 300 ml Dichlormethan gelöst vorgelegt. Unter Rühren und Eiskühlung werden 0,3 mol Carbonsäurechlorid (III) so zugetropft, dass die Temperatur nicht über 5 °C steigt. Man lässt 2 h bei 5 bis 10°C nachrühren und anschließend bei Raumtemperatur über Nacht stehen. Die Reaktionsmischung wird auf Eis gegeben und die wässrige Phase abgetrennt, Man wäscht die organische Phase mit 5 Gew.-%iger Salzsäure, mit gesättigter Natriumchloridlösung, mit 5 Gew.-%iger Soda- und nochmals mit Kochsalzlösung. Die organische Phase wird getrocknet und das Lösemittel unter Vakuum verdampft. Das Rohprodukt wird durch Umkristallisieren oder durch Säulenchromatographie gereinigt.

**Variante C:**

**Allgemeine Synthesevorschrift:**

0,2 mol Hydroxymethyl-pyrazol (II) in 100 ml Essigsäure und 0,4 mol Acetanhydrid werden 24 Stunden lang auf 60 °C erhitzt. Die Reaktionsmischung wird in Eiswasser gegeben und mit 5 Gew.-%iger Natronlauge auf pH 8 gebracht. Es wird mit Chloroform extrahiert, die organische Phase wird mit Wasser gewaschen, getrocknet und einrotiert. Das Rohprodukt wird durch Säulenchromatographie gereinigt.

**Variante D:**

**Allgemeine Synthesevorschrift:**

0,2 mol Hydroxymethyl-pyrazol (II) und 0,2 Mol% 4-Dimethylamino-pyridin (DMAP) werden in 400 ml Dichlormethan gelöst vorgelegt. Man tropft 0,3 mol Carbonsäureanhydrid (IV) und anschließend 0,24 mol Triethylamin zu. Es wird sechs Stunden bei Raumtemperatur nachgerührt. Die Reaktionsmischung wird mit 10 Gew.-%iger Salzsäure, mit gesättigter Natriumchloridlösung, mit Bicarbonat- und nochmals mit Kochsalzlösung extrahiert. Die organische Phase wird getrocknet, das Lösemittel unter Vakuum eingedampft und das Rohprodukt durch Säulenchromatographie oder Umkristallisieren gereinigt.

Die in Tabelle 1 aufgeführten Substanzen wurden nach obigen Synthesevarianten erhalten. Die Strukturen wurden durch IR-, NMR-Spektroskopie und Elementaranalyse gesichert.

Tabelle 1: 1 H-Pyrazol-Derivate der allgemeinen Formel (I)

| Nr. | $R^1$ | $R^2$ | $R^3$ | $R^4$ | Ausb. (%) | Kp (°C) | $^1$H-NMR ($CDCl_3$) $\delta$[ppm] |
|---|---|---|---|---|---|---|---|
| 1 | $CH_3$ | H | H | $CH_3$ | 51 (D) | 149-152 | 2,00 (m, 3 H); 2,20 (s, 1,5 H); 2,30 (s,1,5 H); 5,90 (m, 3 H); 7,40 (d, 1 H); |
| 2 | $CH_3$ | $CH_3$ | H | $CH_3$ | 44 (C) | 241-243 | 1,98 (s, 3 H); 2,00 (s, 3 H) 2,11 (s, 1,5 H); 2,17 (s, 1,5 H); 5,79 (s, 1 H); 5,88 (s, 1 H); 7,26 (m, 1 H); |
| 3 | $CH_3$ | Cl | H | $CH_3$ | 59 (C) | 237-240 | 2,01 (s, 33 H); 2,16 (s, 3 H); 5,80 (s, 1,9 H); 5,89 (s, 0,1 H); 7,52 (s, 1 H); |
| 4 | $CH_3$ | H | H | $C_2H_5$ | 61 (A) | 207-210 | 1,05 (t, 3 H); 2,21 (s, 3 H); 2,28 (q, 2 H); 5,87 (s, 2 H); 6,00 (m, 1H); 7,46 (m, 1 H) |
| 5 | $CH_3$ | H | H | t-$C_4H_9$ | 58 (B) | 232-240 | 1,09 (s, 9 H); 2,15 (s, 3 H); 5,94 (s, 2H); 6,08 (m, 1 H); 7,70 (m, 1H);* |
| 6 | $CH_3$ | H | H | $C_{11}H_{23}$ | 49 (A) | Öl | 0,84 (t, 3 H); 1,21 (m, 18 H); 1,55 (m, 2 H); 2,30 (m, 5 H); 5,90 (s, 1 H); 6,03 (m, 1 H); 7,48 (m, 1 H) |
| 7 | $CH_3$ | Cl | H | $C_{11}H_{23}$ | 57 (A) | Fp: 24-29 | 0,85 (t,3 H); 1,22 (m, 18 H); 1,57 (t, 2 H); 2,21 (s, 3 H); 2,30 (q, 2 H); 5,85 (s, 2 H); 7,56 (s, 1 H) |

(fortgesetzt)

| Nr. | $R^1$ | $R^2$ | $R^3$ | $R^4$ | Ausb. (%) | Kp (°C) | $^1$H-NMR ($CDCl_3$) $\delta$[ppm] |
|---|---|---|---|---|---|---|---|
| 8 | $CH_3$ | H | H | Ph | 60 (A) | Fp: 30-46 | 2,27 (s, 1,1 H); 2,39 (s, 1,9 H); 6,06 (m, 1 H); 6,16 (s , 1,3 H); 6,23 (s, 0,7 H); 7,38-7,63 (m, 4 H); 8,03 (m, 2H) |
| 9 | $CH_3$ | $CH_3$ | H | Ph | 75 (A) | Fp: 62-66 | 1,93 (s, 3 H); 2,16 (s, 2,6 H); 2,36 (s, 0,4 H); 6,09 (s, 1,8 H); 6,19 (s, 0,2 H); 7,35-7,48 (m, 4 H); 7,99 (m, 2 H) |
| 10 | $CH_3$ | Cl | H | Ph | 59 (A) | Fp: 86-88 | 2,21 (s, 3H); 6,09 (s,2 H); 7,37-7,67 (m, 4 H); 8,00 (m, 2 H) |
| 11 | $CH_3$ | H | H | 4-$NO_2$-$C_6H_4$ | 45 (A) | Fp: 100-112 | 2,17 (s, 3 H); 6,16 (s, 1 H); 6,26 (s, 1 H); 7,87 (s, 1 H); 8,16 (d, 2 H ); 8,31 (d, 2 H) * |
| * in DMSO-$d_6$ aufgenommen | | | | | | | |

Ermittlung der nitrifikationshemmenden Wirkung

Die Prüfung der erfindungsgemäßen Pyrazol-Derivate erfolgte in den in Tabelle 2 ausgewiesenen Konzentrationen. Dabei wurde die nitrifikationshemmende Wirkung in der Weise ermittelt, dass 100 g lufttrockener Boden auf 50 Gew.-% der maximalen Wasserkapazität eingestellt wurden. Hierzu wurden jeweils 10 mg Harnstoff-N und die ausgewiesene Wirkstoffmenge unter möglichst homogener Verteilung zugesetzt. Danach werden die so präparierten Gefäße unter standardisierten Bedingungen bei 20°C im dunklen Klimaraum aufbewahrt. Aus der zeitabhängigen Bestimmung des Umsatzes von Ammonium-N zu Nitratstickstoff im Vergleich zur Variante ohne Inhibitor wird die prozentuale Hemmung zum Tag X ermittelt. Der nachfolgend verwendete K-Wert verkörpert ein von den biologischen Schwankungen der Bodenaktivität unabhängigen Wert, da er auf die Wirkung eines Standards, in diesem Fall 3-Methylpyrazol mit einer Inhibitorkonzentration von 0,2 % N-bezogen, durch Quotientenbildung relativiert wird. Der Ermittlung des K-Wertes ist die Berechnung des $t_{50}$-Wertes vorangestellt. Dieser Wert drückt den Zeitpunkt in Tagen aus, an dem die Nitrifikationshemmung für eine einzelne Substanz auf 50 % zurückgegangen ist. Daraus leitet sich der K-Wert wie folgt ab:

$$K = t_{50}\text{-Wert der Testsubstanz}/t_{50}\text{-Wert Standard}$$

Tabelle 2: Nitrikationshemmende Wirkung ausgewählter Verbindungen

| Nr. der Verbindung | Wirkstoffkonzentration ppm | K-Wert |
|---|---|---|
| 1 | 0,2<br>0,5<br>1,0 | 0,7<br>1,1<br>1,3 |
| 2 | 0,2<br>0,5<br>1,0 | 0,9<br>1,3<br>1,4 |
| 3 | 0,2<br>0,5<br>1,0 | 1,3<br>1,3<br>1,3 |
| 4 | 0,5<br>1,0 | 1,1<br>1,2 |
| 5 | 0,5<br>1,0 | 0,9<br>1,2 |

(fortgesetzt)

| Nr. der Verbindung | Wirkstoffkonzentration ppm | K-Wert |
|---|---|---|
| 6 | 0,5<br>1,0 | 0,8<br>1,0 |
| 7 | 0,5<br>1,0 | 1,3<br>1,2 |
| 8 | 0,5<br>1,0 | 1,0<br>1,2 |
| 9 | 0,2<br>0,5<br>1,0 | 0,8<br>1,3<br>1,4 |
| 10 | 0,2<br>0,5<br>1,0 | 1,2<br>1,3<br>1,3 |
| 11 | 0,5<br>1,0 | 0,8<br>1,1 |

K-Werte um den Faktor 1 entsprechen dabei der Wirkung der Standardsubstanz. Unter diesem Aspekt kann Tabelle 2 entnommen werden, dass die erfindungsgemäßen Verbindungen über eine ausgezeichnete nitrifikationshemmende Wirkung verfügen, ohne die für eine praktische Anwendung entgegenstehenden Nachteile des Standards 3-Methylpyrazol wie Flüchtigkeit und Formulierungsunverträglichkeit mit speziell Feststoffdüngern aufzuweisen.

## Patentansprüche

**1.** 1H-Pyrazol-Derivate der allgemeinen Formel (I)

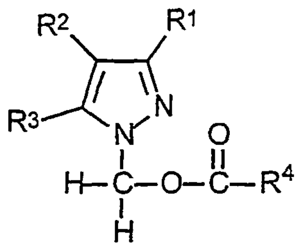

(I)

wobei
$R^1$ für einen $C_1$-$C_4$-Alkylrest oder $C_3$-$C_8$-Cycloalkylrest,
$R^2$ für H, Halogen (F, Cl, Br, I), einen $C_1$-$C_4$-Alkylrest oder $C_3$-$C_8$-Cycloalkylrest,
$R^3$ für H und
$R^4$ für $C_1$-$C_{20}$-Alkyl, $C_3$-$C_8$-Cycloalkyl, $C_6$-$C_{10}$-Aryl oder Alkylaryl mit $C_1$-$C_4$-Alkyl- und $C_6$-$C_{10}$-Arylgruppen steht, wobei die Alkyl- und Arylreste von $R^4$ durch eine Halogen-, Hydroxyl-, Trimethylsilyl-, Amino-, Nitro-, Cyano-, Carbonyl-, Carboxyl- oder eine Carboxyalkylgruppe mit 1 bis 5 C-Atomen substituiert sein können.

**2.** 1H-Pyrazol-Derivate nach Anspruch 1, **dadurch gekennzeichnet, dass** $R^1$ und $R^2$ unabhängig voneinander einen Methylrest darstellen.

**3.** 1H-Pyrazol-Derivate nach Anspruch 1, **dadurch gekennzeichnet, dass** $R^1$ einen Methylrest darstellt und $R^2$ für Chlor oder Wasserstoff steht.

4. Verfahren zur Herstellung der 1H-Pyrazol-Derivate (I) nach den Ansprüchen 1 bis 3, **dadurch gekennzeichnet, dass** man die entsprechenden 1-Hydroxymethyl-pyrazole der allgemeinen Formel (II)

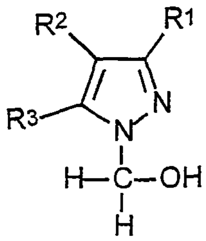

(II)

wobei
$R^1$, $R^2$ und $R^3$ oben genannte Bedeutung besitzen, mit Carbonsäurechloriden $R^4$-CO-Cl,
wobei
$R^4$ die oben genannte Bedeutung besitzt,
in einem organischen Lösemittel unter Zusatz von Basen und gegebenenfalls unter Zusatz eines Acylierungskatalysators reagieren lässt.

5. Verfahren nach Anspruch 4, **dadurch gekennzeichnet, dass** man die Reaktion in einem organischen Lösemittel, ausgewählt aus der Gruppe Halogenkohlenwasserstoffe oder aromatischen Kohlenwasserstoffe, unter Zusatz von 1 bis 100 Mol % eines Acylierungskatalysators wie 4-Dimethylamino-pyridin (DMAP) oder Pyridin sowie ggf. unter Zusatz von 1,0 bis 2 mol einer Base wie Pyridin, Triethylamin oder Kaliumcarbonat mit einem Molverhältnis von 1-Hydroxymethyl-pyrazol zu Carbonsäurechlorid von 1:1,0 bis 1,5 und bei Temperaturen von - 15 bis 100°C durchführt.

6. Verfahren nach Anspruch 4 oder 5. **dadurch gekennzeichnet, dass** man die Reaktion bei - 10 bis 10 °C durchführt.

7. Verfahren nach einem der Ansprüche 4 bis 6, **dadurch gekennzeichnet, dass** man Pyridin als Lösemittel verwendet.

8. Verfahren zur Herstellung der 1H-Pyrazol-Derivate (I) nach den Ansprüchen 1 bis 3, **dadurch gekennzeichnet, dass** man die entsprechenden 1-Hydroxymethyl-pyrazole der allgemeinen Formel (II)

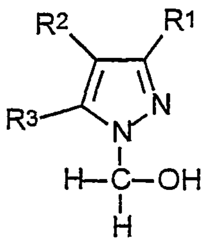

(II)

wobei
$R^1$, $R^2$ und $R^3$ oben genannte Bedeutung besitzen, mit Carbonsäuranhydriden $R^4$CO-O-CO-$R^4$,
wobei
$R^4$ die oben genannte Bedeutung besitzt,
in einem organischen Lösemittel ggf. unter Zusatz von organischen Basen umsetzt.

9. Verfahren nach Anspruch 8, **dadurch gekennzeichnet, dass** man die Reaktion in einem organischen Lösemittel, ausgewählt aus der Gruppe Halogenkohlenwasserstoffe oder aliphatische Ether, unter Zusatz von 0,2 bis 10 Mol % eines Acylierungskatalysators wie 4-Dimethylaminopyridin (DMAP) und/oder molaren Mengen einer organischen Base wie Triethylamin mit einem Molverhältnis von 1-Hydroxymethyl-pyrazol zu Carbonsäureanhydrid von 1 : 1,0 bis 2,5 und bei Temperaturen von - 5 bis 80 °C durchführt.

10. Verfahren nach Anspruch 8, **dadurch gekennzeichnet, dass** Hydroxymethylpyrazol (II) und Carbonsäureanhydrid unter Einsatz der entsprechenden Carbonsäure $R^4$-COOH als Lösemittel bei Temperaturen von 50 bis 100°C zur Reaktion gebracht werden.

11. Verwendung der 1H-Pyrazol-Derivate nach den Ansprüchen 1 bis 3 zur Hemmung bzw. Steuerung der Nitrifikation.

12. Zusammensetzung umfassend die 1H-Pyrazol-Derivate nach den Ansprüchen 1 bis 3 sowie gegebenenfalls weitere übliche Nitrifikationsinhibitoren und/oder Ureasehemmstoffe in Kombination mit festen oder flüssigen ammonium- und/oder amidhaltigen Düngemitteln.

13. Zusammensetzung nach Anspruch 12, **dadurch gekennzeichnet, dass** die 1H-Pyrazol-Derivate sowie die gegebenenfalls weiteren üblichen Nitrifikationsinhibitoren und/oder Ureasehemmstoffe in einer Gesamtaufwandmenge von 0,01 bis 10,0 Gewichtsprozent berechnet auf den reduzierten Stickstoffanteil des amid- und/oder ammoniumhaltigen Düngemittels eingesetzt werden.

14. Zusammensetzung nach Anspruch 13, **dadurch gekennzeichnet, dass** die 1H-Pyrazol-Derivate in einer Aufwandmenge von 0,1 bis 3,0 Gewichtsprozent eingesetzt werden.

15. Zusammensetzung nach einem der Ansprüche 12 bis 14, **dadurch gekennzeichnet, dass** die 1H-Pyrazol-Derivate allein oder im Gemisch mit weiteren Zusatzstoffen in die Düngemittelschmelze oder -slurry während des Formgebungsprozesses eingebracht worden sind und auf diese Weise homogen im Dünger verteilt vorliegen.

16. Zusammensetzung nach einem der Ansprüche 12 bis 14, **dadurch gekennzeichnet, dass** die 1H-Pyrazol-Derivate ggf. mit weiteren Zusatzstoffen in Form einer Lösung auf die Oberfläche der Düngemittelgranulate aufgebracht worden sind und anschließend das Lösemittel durch Trocknung entfernt wurde.

## Claims

1. 1H-pyrazole derivatives with the general formula (I)

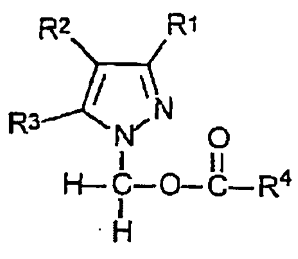

(I)

where
$R^1$ represents a $C_1$-$C_4$-alkyl moiety or $C_3$-$C_8$-cycloalkyl moiety,
$R_2$ an H, halogen (F, Cl, Br, I), a $C_1$-$C_4$-alkyl moiety or $C_3$-$C_8$-cycloalkyl moiety,
$R^3$ H, and
$R^4$ $C_1$-$C_{20}$-alkyl, $C_3$-$C_8$-cycloalkyl, $C_6$-$C_{10}$-aryl or alkylaryl with $C_1$-$C_4$-alkyl and $C_6$-$C_{10}$-aryl groups, it being possible to substitute the alkyl and aryl moieties of $R^4$ with a halogen, hydroxyl, trimethylsilyl, amino, nitro, cyano, carbonyl, carboxyl or a carboxylalkyl group with 1 to 5 C-atoms.

2. 1H-pyrazole derivatives according to Claim 1, **characterised in that** $R^1$ and $R^2$, independently of one another, represent a methyl moiety.

3. 1H-pyrazole derivatives according to Claim 1, **characterised in that** $R^1$ represents a methyl moiety and $R^2$ represents chlorine or hydrogen.

4. A process for producing 1 H-pyrazole derivatives (I) according to Claims 1 to 3, **characterised in that** the corresponding 1-hydroxymethyl-pyrazoles with the general formula (II)

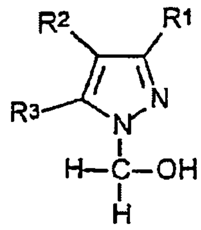

(II)

where
$R^1$, $R^2$ and $R^3$ have the meaning specified above,
with carboxylic acid chlorides $R^4$-CO-Cl,
where
$R^4$ has the meaning specified above,
are allowed to react in an organic solvent to which bases, and optionally an acylation catalyst, are added.

**5.** The process according to Claim 4, **characterised in that** the reaction is implemented in an organic solvent, chosen from the halogen hydrocarbon group or aromatic hydrocarbon group, with the addition of 1 to 100 mole % of an acylation catalyst such as 3-dimethylaminopyridine (DMAP) or pyridine and optionally with the addition of 1.0 to 2 mole of a base such as pyridine, triethylamine or potassium carbonate with a mole ratio of 1-hydroxymethyl-pyrazole to carboxylic acid chloride of 1 : 1.0 to 1.5 and at temperatures of -15 to 100°C.

**6.** The process according to Claim 4 or 5, **characterised in that** the reaction is implemented at -10 to 10°C.

**7.** The process according to any of Claims 4 to 6, **characterised in that** pyridine is used as a solvent.

**8.** A process for producing the 1 H-pyrazole derivatives (I) according to Claims 1 to 3, **characterised in that** the corresponding 1-hydroxymethyl-pyrazoles with the general formula (II)

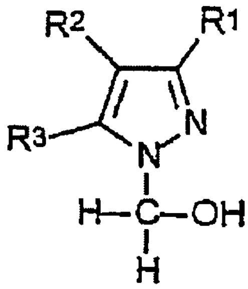

(II)

where
$R^1$, $R^2$ and $R^3$ have the meaning specified above,
with carboxylic acid anhydrides $R^4$CO-O-CO-$R^4$,
where
$R^4$ has the meaning specified above,
is converted in an organic solvent, optionally with the addition of organic bases.

**9.** The process according to Claim 8, **characterised in that** the reaction is implemented in an organic solvent chosen from the halogen hydrocarbon or aliphatic ether group with the addition of 0.2 to 10 mole % of an acylation catalyst

such as 4-dimethylaminopyridine (DMAP) and/or molar quantities of an organic base such as triethylamine with a mole ratio of 1-hydroxymethyl-pryrazole to carboxylic acid anhydride of 1 : 1.0 to 2.5 and at temperatures of -5 to 80°C.

**10.** The process according to Claim 8, **characterised in that** hydroxymethylpyrazole (II) and carboxylic acid anhydride are brought to a reaction using the corresponding carboxylic acid $R^4$-COOH as a solvent at temperatures of 50 to 100°C.

**11.** The use of 1 H-pyrazole derivatives according to Claims 1 to 3 in order to inhibit or control nitrification.

**12.** A composition comprising the 1 H-pyrazole derivatives according to Claims 1 to 3 and optionally further conventional nitrification inhibitors and/or urease inhibitors in combination with solid or liquid fertilisers containing ammonium and/or amide.

**13.** The composition according to Claim 12, **characterised in that** the 1 H-pyrazole derivatives and optionally the further conventional nitrification inhibitors and/or urease inhibitors are used in a total application quantity of 0.01 to 10.0 percent by weight calculated according to the reduced nitrogen portion of the fertiliser containing amide and/or ammonium.

**14.** The composition according to Claim 13, **characterised in that** the 1 H-pyrazole derivatives are used in an application quantity of 0.1 to 3.0 percent by weight.

**15.** The composition according to any of Claims 12 to 14, **characterised in that** the 1 H-pyrazole derivatives have been introduced on their own or in a mixture with further additives into the fertiliser liquefied mass or slurry during the forming process and in this way are present homogeneously distributed in the fertiliser.

**16.** The composition according to any of Claims 12 to 14, **characterised in that** the 1 H-pyrazole derivatives, optionally with further additives in the form of a solution, have been applied over the surface of the fertiliser granulates, and the solvent was then removed by drying.

**Revendications**

**1.** Dérivés de 1 H-pyrazole correspondant à la formule générale (I)

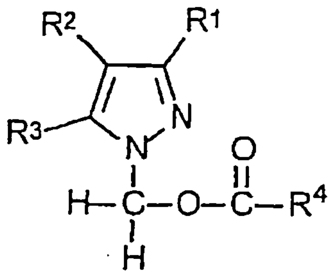

(I)

dans laquelle
$R^1$ représente un reste alkyle en $C_1$-$C_4$ ou un reste cycloalkyle en $C_3$-$C_8$,
$R^2$ représente un atome d'hydrogène, un atome d'halogène (F, Cl, Br, I), un reste alkyle en $C_1$-$C_4$ ou un reste cycloalkyle en $C_3$-$C_8$,
$R^3$ représente un atome d'hydrogène, et
$R^4$ représente un alkyle en $C_1$-$C_{20}$, un cycloalkyle en $C_3$-$C_8$, un aryle en $C_6$-$C_{10}$ ou un alkylaryle présentant des groupes alkyle en $C_1$-$C_4$ et aryle en $C_6$-$C_{10}$, les restes alkyle et aryle de $R^4$ pouvant être substitués par un groupe halogéno, hydroxyle, triméthylsilyle, amino, nitro, cyano, carbonyle, carboxyle ou un groupe carboxyalkyle comportant de 1 à 5 atomes de carbone.

**2.** Dérivés de 1H-pyrazole selon la revendication 1, **caractérisés en ce que** $R^1$ et $R^2$ représentent indépendamment

l'un de l'autre un reste méthyle.

3. Dérivés de 1H-pyrazole selon la revendication 1, **caractérisés en ce que** $R^1$ représente un reste méthyle et $R^2$ représente un atome de chlore ou d'hydrogène.

4. Procédé de production des dérivés de 1H-pyrazole (I) selon les revendications 1 à 3, **caractérisé en ce que** l'on fait réagir les 1-hydroxyméthylpyrazoles appropriés correspondant à la formule générale (II)

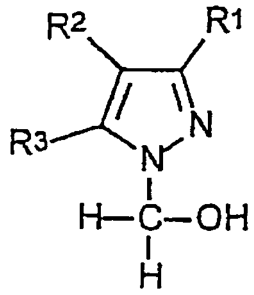

(II)

dans laquelle
$R^1$, $R^2$ et $R^3$ ont les significations ci-dessus,
avec des chlorures d'acide carboxylique $R^4$-CO-Cl,
dans lesquels $R^4$ a la signification ci-dessus,
dans un solvant organique avec ajout de bases et éventuellement avec ajout d'un catalyseur d'acylation.

5. Procédé selon la revendication 4, **caractérisé en ce que** l'on conduit la réaction dans un solvant organique, choisi dans le groupe constitué par les hydrocarbures halogénés ou les hydrocarbures aromatiques, avec ajout de 1 à 100 % en mol d'un catalyseur d'acylation tel que la 4-diméthylaminopyridine (DMAP) ou la pyridine ainsi éventuellement qu'avec un ajout de 1,0 à 2 mol d'une base telle que la pyridine, la triéthylamine ou le carbonate de potassium dans un rapport molaire entre le 1-hydroxyméthylpyrazole et le chlorure d'acide carboxylique allant de 1 : 1,0 à 1,5 et à des températures allant de -15 à 100 °C.

6. Procédé selon la revendication 4 ou 5, **caractérisé en ce que** l'on conduit la réaction entre - 10 et 10°C.

7. Procédé selon l'une des revendications 4 à 6, **caractérisé en ce que** le solvant utilisé est la pyridine.

8. Procédé de production des dérivés de 1H-pyrazole (I) selon les revendications 1 à 3, **caractérisé en ce que** l'on fait réagir les 1-hydroxyméthylpyrazoles appropriés correspondant à la formule générale (II)

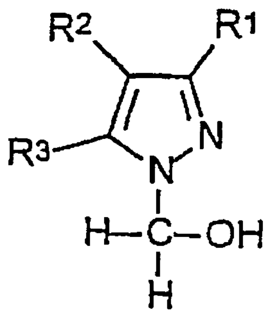

(II)

dans laquelle
$R^1$, $R^2$ et $R^3$ ont la signification ci-dessus,

avec des anhydrides de l'acide carboxylique $R^4CO-O-CO-R^4$,
dans lesquels
$R^4$ a la signification ci-dessus,
dans un solvant organique avec ajout éventuel de bases organiques.

**9.** Procédé selon la revendication 8, **caractérisé en ce que** l'on conduit la réaction dans un solvant organique, choisi dans le groupe constitué par les hydrocarbures halogénés ou les éthers aliphatiques, avec ajout de 0,2 à 10 % en mol d'un catalyseur d'acylation tel que la 4-diméthylaminopyridine (DMAP) et/ou des quantités molaires d'une base organique telle que la triéthylamine dans un rapport molaire entre le 1-hydroxyméthylpyrazole et l'anhydride d'acide carboxylique allant de 1 : 1,0 à 2,5 et à des températures allant de -5 à 80 °C.

**10.** Procédé selon la revendication 8, **caractérisé en ce que** l'hydroxyméthylpyrazole (II) et l'anhydride d'acide carboxylique sont mis à réagir en utilisant comme solvant l'acide carboxylique approprié $R^4$-COOH et à des températures allant de 50 à 100 °C.

**11.** Utilisation des dérivés de 1H-pyrazole selon les revendications 1 à 3 pour l'inhibition ou le contrôle de la nitrification.

**12.** Composé renfermant les dérivés de 1H-pyrazole selon les revendications 1 à 3 ainsi éventuellement que d'autres inhibiteurs de la nitrification et/ou inhibiteurs de l'uréase usuels, en combinaison avec des engrais solides ou liquides contenant de l'ammonium et/ou des amides.

**13.** Composé selon la revendication 12, **caractérisé en ce que** les dérivés de 1H-pyrazole ainsi que les éventuels autres inhibiteurs de la nitrification et/ou inhibiteurs de l'uréase usuels sont utilisés dans une quantité d'application totale allant de 0,01 à 10,0 pour cent en poids par rapport à la proportion d'azote réduit de l'engrais contenant des amides et/ou de l'ammonium.

**14.** Composé selon la revendication 13, **caractérisé en ce que** les dérivés de 1H-pyrazole sont utilisés dans une quantité d'application allant de 0,1 à 3,0 pour cent en poids.

**15.** Composé selon l'une des revendications 12 à 14, **caractérisé en ce que** les dérivés de 1H-pyrazole sont employés seuls ou en mélange avec d'autres additifs dans la coulée d'engrais ou la bouillie d'engrais au cours du processus de formage et se présentent ainsi de manière homogène dans le fertilisant.

**16.** Composé selon l'une des revendications 12 à 14, **caractérisé en ce que** les dérivés de 1H-pyrazole sont appliqués à la surface des granulés fertilisants sous la forme d'une solution impliquant éventuellement d'autres additifs, avant que les solvants ne soient éliminés par séchage.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- US 3635690 A
- US 4969946 A
- US 4523940 A
- EP 166420 A
- JP OS7104135 A
- US 3697244 A
- DE OS2745833 A
- EP 508191 A
- DD 292232
- DE OS4018395 A
- EP 160804 A
- DE OS3704359 A
- SU 1470732
- DE OS4446194 A
- EP 808297 A
- EP 529473 A
- WO 9805607 A
- SU 1470737 A
- DE OS10117821 A
- DE OS10135423 A


### In der Beschreibung aufgeführte Nicht-Patentliteratur


- *J. Hüttel Chem. Ber.,* 1952, vol. 85